# EUROPEAN PATENT APPLICATION

(11) **EP 4 226 862 A1**
(43) Date of publication of application: **16.08.2023**
(21) Application number: 22156101.2
(22) Date of filing: 10.02.2022
(51) Int. Cl.: A61B 6/04, G01R 33/28, G16H 40/63

(54) **HAPTIC FEEDBACK ASSISTED POSITIONING OF IMAGING ACCESSORY**

(71) Applicant: Koninklijke Philips N.V., 5656 AG Eindhoven (NL)
(72) Inventor: LEUSSLER, Christoph Günther, Eindhoven (NL); AMTHOR, Thomas Erik, Eindhoven (NL)
(74) Representative: Philips Intellectual Property & Standards

(57) **Abstract**

The invention relates to an imaging accessory positioning system (11) and method for medical imaging. Haptic feedback (151) is used to assist a user (16) with positioning an imaging accessory (12) relative to a reference position (122). A position sensor (13) measures the position (121) of the imaging accessory (12) and provides a position signal (131). A processing unit (14) derives the position (121) of the imaging accessory (12) from the position signal (131), computes a feedback signal (141) based on the deviation between the position (121) of the imaging accessory (12) and the reference position (122), and provides the feedback signal (141) to a haptic feedback unit (15). The haptic feedback unit (15) generates haptic feedback (151) to assist the user (16). In one embodiment, the reference position (122) relative to a patient is automatically determined.

## Description

### FIELD OF THE INVENTION

The invention relates to the field of medical imaging, and more specifically to assisted positioning of imaging accessories for such imaging.

### BACKGROUND OF THE INVENTION

European patent application EP3618079A1 describes a system for controlling operation of an imaging or therapy apparatus. The system comprises an interface for receiving a pain measurement signal as measured by one or more sensors in relation to an anatomic part of the patient i) being imaged in an imaging procedure by the imaging apparatus or ii) being under therapy in a therapy procedure delivered by the therapy device, whilst the part is held in an adjustable fixation device. According to EP3618079A1, the system may allow autonomous imaging or therapy where the presence of a user may not be necessary at all times during the imaging operation.

High patient throughput is crucial for many medical imaging facilities. Fully or partly automated imaging workflow steps may increase throughput and reduce operational costs. Reduced presence of staff near to patients may also limit infection risks.

Some imaging procedures require additional equipment to be placed on or adjacent to the patient before or during imaging. E.g., radio frequency coils must be correctly positioned in the case of magnetic resonance imaging to ensure proper imaging quality and patient safety. The patient may be lying down on a bed when positioning the accessory. Accurate and timely positioning of such imaging accessories can be difficult for the patient or a person helping the patient.

### SUMMARY OF THE INVENTION

It is, inter alia, an object of the invention to assist a user with positioning of imaging accessories.

The invention is defined by the independent claims. Advantageous embodiments are defined in the dependent claims.

Embodiments of the invention may provide one or more of the following advantages. It would be advantageous to provide automated feedback to the user during positioning of the imaging accessory to assist the user. It would also be advantageous if the feedback is adapted based on where the imaging accessory is currently positioned compared to an intended reference position. It would also be desirable to provide feedback to the user, which does not require the user to see the accessory e.g., when lying down, or requires use of additional hardware for visual instructions. Furthermore, it would be advantageous if the feedback is fast, intuitive and easy to respond to.

Less operator dependency in positioning of imaging accessories may also provide objectivity to the medical imaging procedures. At the same time, patient engagement and patient experience are important factors for medical imaging facilities and may be improved by user guidance.

In a first aspect of the invention an imaging accessory positioning system is presented. The imaging accessory positioning system includes: an imaging accessory to be positioned relative to a reference position; a position sensor that measures the position of the imaging accessory and provides a position signal; a processing unit configured to receive, store and/or determine the reference position, derive the position of the imaging accessory from the position signal, compare the position of the imaging accessory to the reference position, and compute a feedback signal based on the deviation between the position of the imaging accessory and the reference position; a haptic feedback unit that provides haptic feedback to a user in response to the feedback signal. The haptic feedback assists the user with positioning of the imaging accessory relative to the reference position.

Haptic feedback may be very intuitive and fast to respond to. Such feedback may be experienced as direct, easy and/or with low complexity. Speed and/or accuracy of positioning medical image accessories by staff and/or patients under stress may therefore be improved and/or mistakes may be avoided.

The imaging accessory is an accessory that needs to be positioned for medical imaging. It is advantageous that embodiments of the invention are applicable to various imaging accessories. Examples include an RF coil assembly or an RF coil connector for magnetic resonance imaging, patient protection for X-ray imaging, positioning of vital signs sensors for use during imaging, positioning of accessories for image guided therapy etc.

The intended reference position for the imaging accessory may be defined before initiating positioning of the imaging accessory and/or during positioning of the imaging accessory. The reference position may be defined relative to a frame of reference such as, but not limited to, the patient, a patient bed, another imaging accessory, or an imaging system. This means that the reference position may not be static and can move with the frame of reference.

The user is the person positioning the imaging accessory. In cases where the patient to be imaged is positioning the accessory himself/herself, the user is the patient.

In a preferred embodiment, the haptic feedback unit is located on or integrated with the imaging accessory. This has the advantage that the user can thereby touch the haptic feedback unit and get haptic feedback directly when holding or otherwise being in contact with the imaging accessory.

In another preferred embodiment, the haptic feedback unit on the imaging accessory has one or multiple touch sensors and/or inertial sensors, to detect touching and/or movement. This may allow for more precise and/or intuitive haptic feedback and/or accurate positioning assistance of the imaging accessory.

A touch sensor may be e.g. a capacitive touch sensor, a resistive touch sensor or an optical touch sensor. The touch sensor is a sensor able to sense e.g., if there is physical contact with the sensor, close proximity to the sensor and/or the amount of force with which the sensor is touched.

An inertial sensor, such as an inertial measurement unit, is a sensor that senses rotational motion, translational motion and/or orientation. The measurement unit may contain one or more of e.g. accelerometers, gyroscopes and/or magnetometers.

In preferred embodiments, the haptic feedback unit on the imaging accessory detects at least one position where the user touches the unit and provides feedback at that position. It is advantageous that haptic feedback is generated where the user is in contact with the haptic feedback unit in order to provide directed and intuitive feedback to the user.

In another preferred embodiment, a characteristic of the haptic feedback is automatically adapted to a stage of an imaging workflow. It may be advantageous for automated workflows and/or autonomous imaging if the system can adapt the feedback that is provided to a user in order to match the current stage of the imaging workflow. By way of non-limiting examples, the type, intensity, location, duration and/or activation of haptic feedback may be automatically adapted.

In preferred embodiments, the position sensor is a LIDAR or RADAR or 3D camera sensor that can generate an image of the imaging accessory and the patient.

As an example, the position sensor may be located on or in proximity to the imaging apparatus and provide images of the patient and imaging accessory when the patient is placed on a patient bed in front of or inside the imaging apparatus. In this way the position sensor can provide contactless measurements of both the position of the imaging accessory and the reference frame relative to which the accessory should be positioned, such as the patient. This also gives the possibility to adapt the feedback to the user in response to movement of the imaging accessory and the reference position.

In another preferred embodiment, the processing unit uses images from the position sensor to automatically determine the preferred reference position of the imaging accessory relative to the patient. This has the possible advantage of improved autonomous imaging and/or patient safety.

According to a second aspect of the invention, there is provided a medical imaging system comprising the imaging accessory positioning system.

According to a third aspect of the invention, there is provided a method of positioning an imaging accessory for medical imaging. The method comprises: receiving a position signal from a position sensor configured to measure a position of the imaging accessory; processing the position signal to compare the position of the imaging accessory to a reference position; computing a feedback signal based on the deviation of the position of the imaging accessory and the reference position; sending the feedback signal to a haptic feedback unit. The feedback signal is configured to trigger the haptic feedback unit to generate haptic feedback to a user. The haptic feedback triggered by the feedback signal is configured to assist the user with positioning of the imaging accessory relative to the reference position.

In embodiments, the processed position signal includes an image of the imaging accessory and the patient. This allows for further processing, such as but not limited to identification of the imaging accessory, patient type etc.

In embodiments, the reference position is automatically determined relative to the position of the patient. This may allow for autonomous imaging workflows and/or improved speed of patient preparation etc.

According to another aspect of the invention, there is provided a computer program element, which, when being executed by at least one processing unit, is adapted to cause the processing unit to perform the steps of the third aspect of the invention and any related embodiments.

According to another aspect of the invention, there is provided a computer readable medium having stored thereon the above-mentioned program element.

According to a fourth aspect of the invention, there is provided an imaging accessory configured to be used in an imaging accessory positioning system for medical imaging.

These and other aspects of the invention will be apparent from and elucidated with reference to the embodiments described hereinafter.

### BRIEF DESCRIPTION OF THE DRAWINGS

Fig. 1 shows a schematic of an imaging accessory positioning system in accordance with an embodiment of the invention.
Fig. 2 shows a schematic of an imaging accessory positioning system in accordance with another embodiment of the invention.
Fig. 3 shows a flowchart representing a method of positioning an imaging accessory in accordance with an embodiment of the invention.
Fig. 4. shows a flowchart representing a method of positioning an imaging accessory in accordance with an embodiment of the invention.

### DESCRIPTION OF EMBODIMENTS

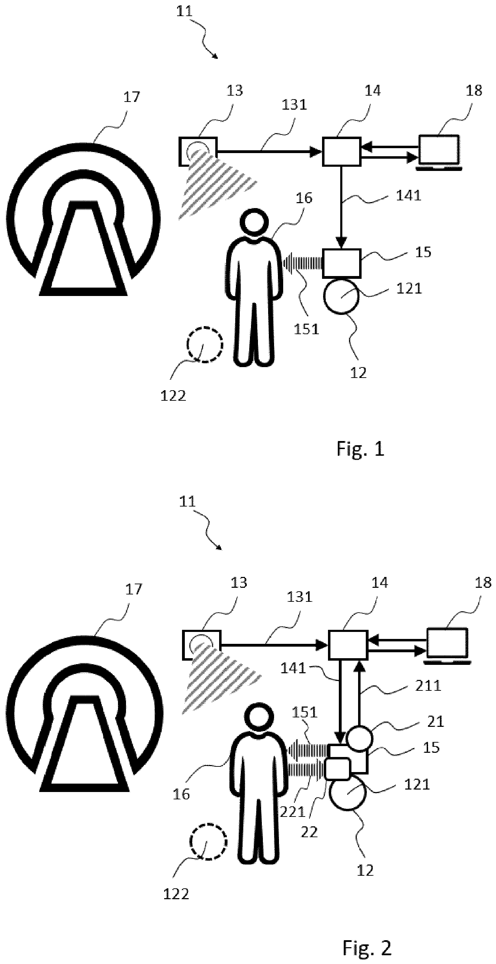

Fig. 1 illustrates an example embodiment of the invention. An imaging accessory positioning system 11 is configured to assist a user 16 with positioning of an imaging accessory 12. The imaging accessory 12 needs to be correctly placed in a reference position 122 so that a patient can be imaged with a medical imaging apparatus 17. In this example, the user 16 is the patient to be imaged and therefore wants to position the imaging accessory 12 him/herself relative to his/her body as part of the imaging workflow. However, it can also be envisioned that the user 16 is a different person helping the patient set up for imaging. Many different imaging accessories 12 that need correct positioning can be envisioned, such as an RF coil assembly for magnetic resonance imaging, patient protection gear for X-ray imaging, positioning of vital signs sensors for use during imaging, positioning of accessories for image guided therapy etc.

The position sensor 13 is e.g., a RADAR, LIDAR or 3D camera sensor. The position sensor 13 may also be another similar sensor that can determine a position of an object in three or at least two dimensions from a large or small distance. Preferably the measurement is contactless. The position sensor 13 measures the position 121 of the imaging accessory 12 and sends a position signal 131 to a processing unit 14. The position sensor may also sense other positions such as positions of the user 16, and other objects in its field of view.

A preferred reference position 122 is known, e.g., predefined by an operator or the user 16 via a User Interface 18, or automatically determined from information measured by the position sensor 13.

In the latter case, the processing unit 14 is e.g. configured to automatically identify the imaging accessory 12 and where it should be placed relative to the patient, based on images from the position sensor 13, showing the patient and the imaging accessory 12. It is also possible that the imaging accessory is defined by an operator or the user 16 via the User Interface 18 and that the processing unit 14 based on that information and images from the position sensor 13 automatically determines the reference position 122. In one example, the processing unit 14 can be trained with machine learning to identify preferred reference positions for multiple imaging accessory 12 and/or patient types in various use cases.

When both the current position 121 of the imaging accessory 12 and the reference position 122 are identified, the processing unit 14 compares the positions, determines how to decrease the distance between the positions and generates a feedback signal 141 to a haptic feedback unit 15.

In the example illustrated in Fig.1, the haptic feedback unit 15 is located on or integrated with the imaging accessory 12, such that the user touches the haptic feedback unit 15 when holding the imaging accessory 12.

Based on the feedback signal 141, the haptic feedback unit 15 generates haptic feedback 151 to assist the user 16 with positioning the imaging accessory 12 closer to the reference position 122. Haptics or haptic feedback may be described as creating an experience of touch or tactile sensation by applying stimuli such as forces, vibrations, pressure waves, surface changes, and/or motions. Haptic feedback 151 may preferably be provided to the user 16 at positions where the user 16 is in contact or close proximity with the haptic feedback unit 15.

As an example, the haptic feedback unit 15 on the imaging accessory 12 may have predefined areas to touch the unit. Such as pre-defined finger positions, where haptic feedback 151 is provided.

As another example, the user 16, being the patient, may put the haptic feedback unit 15 integrated with an imaging accessory 12 in contact with a body part where the imaging accessory 12 is to be more accurately positioned. Such as on the torso, head, arm or leg. Haptic feedback 151 at a patient facing surface of the haptic feedback unit 15 in contact with the said body part will in this example be felt by the patient to support further positioning.

The haptic feedback 151 is configured to be interpreted as movement commands by the user 16 and thereby help the user 16 to correctly position the imaging accessory 12.

Non-limiting examples of such movement commands include vibrations or other forces on one side and/or to a limited area of a body part to stimulate movement in a specific direction, gradients in the haptics to stimulate rotation, pulsed signals to communicate progress etc. Many other such combinations of movement commands and haptic feedback configurations can be envisioned to enable intuitively assisted positioning.

To make sure that the imaging accessory 12 is positioned correctly in three dimensions, correctly rotated and so on, the reference position 122 may be defined at one or multiple points in three-dimensional space. Depending on the shape of the imaging accessory 12 and need for accuracy in placement, the reference position can be e.g., one reference point at the center of gravity of where the imaging accessory 12 is intended to be placed or at multiple points of a virtual surface or forming a virtual volume to accurately determine placement of unsymmetric geometries.

The feedback signal 141 from the processing unit 14 is continuously updated so that the haptic feedback unit 15 provides updated haptic feedback 151 to the user 16 while the imaging accessory 12 is moving. In this way, the system is responsive to the current position and movement of the imaging accessory 12 and thereby adjusts to assist the user 16 to intuitively perform the correct positioning. The feedback signal is updated at least every 10 to 60 seconds, preferably every 1 to 10 seconds and even more preferably more often than once per second.

Communication between the different elements of the imaging accessory positioning system can be wireless or wired. Wireless digital communication such as via UMTS, LTE, 5G, WiFi, Bluetooth, Zigbee, Ultrawideband or similar is preferred in many use cases. In other examples, e.g. optical fiber technology may be advantageous. Power may be provided via e.g., power cables or batteries. It may be advantageous to reduce the number of wires connected to imaging accessories in order to increase patient comfort and/or reduce disturbance to the medical image quality caused by cable interference during imaging. In some circumstances, such as close to a high voltage X-ray generator or high magnetic fields in a magnetic resonance imaging environment, it can be envisioned that special shielding of system components may be required.

The position sensor 13 may be located on the imaging apparatus 17, on a wall or a sealing of the room, or at another location wherefrom the position of the imaging accessory 12 may be determined. The processing unit 14 may be located in proximity to or integrated with the position sensor 13, the imaging accessory 12, the haptic feedback unit 15 or the imaging apparatus 17. The processing unit 14 may also be located separately in the same room or outside, such as at a workstation. The User Interface 18 may have a wired or wireless connection to the processing unit 14.

It should be noted that although the purpose of positioning the imaging accessory 12 is for medical imaging, the medical imaging apparatus 17 itself is not necessarily required to be close to the positioning system 11. It can be envisioned that preparation of the patient with positioning of the imaging accessory 12 can take place in a different room from the medical imaging apparatus 17.

Referring back to Fig. 1, the imaging accessory 12 may also be a cable assembly that needs to be positioned correctly to connect with another accessory or at another location, such as relative to the patient bed. By way of example, in another embodiment the imaging accessory 12 is an RF coil cable assembly that needs to be positioned at the reference position 122 to connect with an RF coil.

New patient bed technology is equipped with many connection points. The proposed system and method assist the user 16 to connect a cable assembly at the correct connection point with respect to safe cable routing, mechanical robustness and feasible cable length. In this embodiment, the reference position 122 is thus not necessarily defined relative to the patient but to another object such as the bed or another imaging accessory.

The positioning of the imaging accessory 12, here an RF coil cable assembly or similar, is otherwise comparable to the previous example. Haptic feedback 151 assists the user 16 holding the RF cable assembly with the intended movement of the cable assembly. It is possible that multiple sequential reference positions 122 along a trajectory are preferred in order to guide the user 16 with positioning the RF coil cable assembly to the final position while achieving the correct cable routing.

Fig. 2 illustrates another embodiment of the invention. Similar to the example in Fig. 1, the imaging accessory positioning system 11 in Fig.2 is configured to assist a user 16 with positioning of an imaging accessory 12. The imaging accessory 12 needs to be correctly placed in a reference position 122 so that a patient can be imaged with a medical imaging apparatus 17. The position sensor 13 measures the position of the imaging accessory 12 and sends a position signal 131 to a processing unit 14. A preferred reference position 122 is known. When both the current position 121 of the imaging accessory 12 and the reference position 122 are identified, the processing unit 14 compares the positions, determines how to decrease the distance between the positions and generates a feedback signal 141 to a haptic feedback unit 15. Also in this example, the haptic feedback unit 15 is located on or integrated with the imaging accessory 12, such that the user touches the haptic feedback unit 15 when holding the imaging accessory 12.

In Fig 2, the haptic feedback unit 15 contains at least one inertial sensor 21. Based on measurements of e.g. rotational and translational movement and/or orientation, the inertial sensor generates a movement signal 211 that is communicated with the processing unit. The movement signal, in addition to and in parallel to information from the position signal 131, allows the processing unit to accurately monitor the movement and orientation of the imaging accessory 12, to which the haptic feedback unit 15 is attached, and if needed adapt the feedback signal 141.

Based on the feedback signal 141, the haptic feedback unit 15 generates haptic feedback 151 to assist the user 16 with positioning the imaging accessory 12 closer to the reference position 122.

In Fig 2, the haptic feedback unit 15 also contains at least one touch sensor 22. The touch sensor detects a touch signal 221 from the user 16 and can therefore measure if and/or where the user 16 touches the haptic feedback unit 15. With multiple sensors, e.g. in an array, the touch signal 221 also indicates the current location of where the user touches the haptic feedback unit 15. By providing haptic feedback 151 at the locations where the user 16 touches the haptic feedback unit 15, a more precise and intuitive feedback to the user can be achieved.

The haptic feedback unit 15 may be configured to process the touch signal 221 measured by the touch sensor 22 and potentially adapt when and where the haptic feedback 151 is provided to the user 16.

In another example, not illustrated in the figure, the measurement by the touch sensor 22 is communicated with the processing unit 14 and the feedback signal 141 may be adjusted accordingly.

It can be envisioned that, in embodiments, an imaging accessory positioning system 11 can be used in combination with multiple and/or different imaging accessories 12. It is foreseeable that imaging accessories 12 may be sold separately and/or may be used with the system 11 in a plug & play fashion. Such an imaging accessory 12 may be integrated with a haptic feedback unit 15 and/or configured to connect with a haptic feedback unit 15.

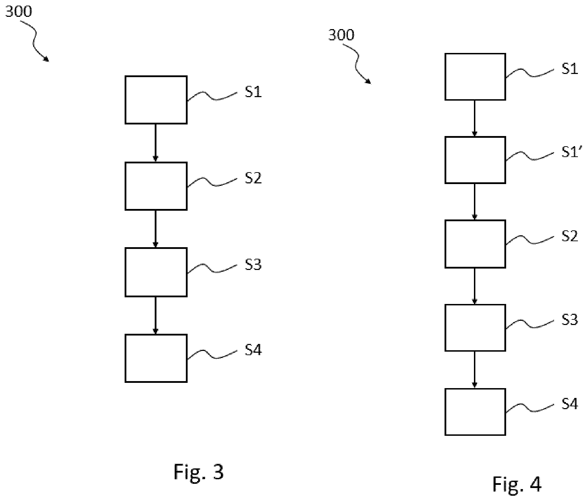

Fig. 3 illustrates an embodiment of a method 300 of positioning an imaging accessory 12 for medical imaging. The method 300 may comprise the following steps:
Receiving S1 a position signal 131 from a position sensor 13 configured to measure a position 121 of the imaging accessory 12.
Processing S2 the position signal 131 to compare the position 121 of the imaging accessory 12 to a reference position 122.
Computing S3 a feedback signal 141 based on the deviation of the position 121 of the imaging accessory 12 and the reference position 122.
Sending S4 the feedback signal 141 to a haptic feedback unit 15.

Moreover, the feedback signal 141 is configured to trigger the haptic feedback unit 15 to generate haptic feedback 151 to a user 16. The haptic feedback 151 is configured to assist the user 16 with positioning of the imaging accessory 12 relative to the reference position 122.

In embodiments, the processed position signal 131 includes at least one image of the imaging accessory 12 and a patient.

Fig. 4 illustrates another embodiment of the method 300 of positioning an imaging accessory 12 for medical imaging. In this example, the processed position signal 131 includes at least one image of the imaging accessory 12 and a patient. In a step S1' the reference position 122 automatically determined relative to the patient. For example, trained machine learning algorithms may be leveraged to identify preferred reference positions for multiple imaging accessory 12 and/or patient types in various use cases.

It should be noted that the above-mentioned embodiments illustrate rather than limit the invention, and that those skilled in the art will be able to design many alternative embodiments without departing from the scope of the appended claims. By way of example, the embodiments described here refer to positioning before imaging takes place, but additional embodiments can also be envisioned where the user 16 is assisted to adjust positioning of imaging accessories 12 during medical imaging, e.g., with the position sensor 13 located or directed inside the bore of an imaging system.

In embodiments, one or many characteristics of the haptic feedback 151 may be automatically adapted during an imaging workflow to match the particular stage of the workflow. As an example, haptic feedback 151 may be generated in response to the position 121 of the imaging accessory 12 during the period when the patient on an imaging apparatus bed prepares for imaging. When the imaging accessory 12 is in the correct reference position 122 a timestamp is created in the system, the patient is notified, and a next stage of the automated workflow begins. During a next stage of the automated workflow, such as the bed moving into the bore of the imaging apparatus 17, haptic feedback 151 may be automatically deactivated. It may be advantageous if the patient does not try correcting the position 121 of the imaging accessory 12 during this part of the workflow, since the bed is moving. Therefore, in this example no haptic feedback 151 is generated by the haptic feedback unit 15 during movement of the bed into the bore, even if the system detects a position change of the imaging accessory 12. Inside the bore, a new timestamp is created, and a following stage of the automated workflow begins, such as specific imaging protocols. Again the characteristic of haptic feedback 151 may be adapted in a pre-defined manner to support the patient during this stage of the workflow. It is conceivable that during imaging the patient needs to remain still, but the imaging accessory 12 needs to be correctly positioned at all times. It may therefore be advantageous if aspects such as the threshold to activate haptic feedback 151 and/or intensity of the feedback is different compared to during other parts of the workflow. Many different combinations of workflow stages and pre-defined characteristics of generated haptic feedback 151 can be envisioned.

By way of non-limiting examples, the type, intensity, location, duration, threshold and/or activation of haptic feedback 151 with the imaging accessory positioning system 11 may be automatically adapted to match each stage of an automated imaging workflow. Examples of workflow timestamps to indicate a stage of a workflow may include 'patient is in the preparation room', 'patient is on the imaging table', 'imaging accessory is being positioned on the patient', 'imaging accessory is fixed', 'imaging accessory is activated', 'patient is ready to be scanned', 'patient is moving into the bore of an imaging apparatus', 'patient is being imaged with protocol A' etc.

In embodiments, the haptic feedback unit 15 may be located in a bed and/or mattress on which the patient lies. Haptic feedback may in such an example be provided to the patient via the surfaces where the patient is in contact with the bed or mattress.

In other embodiments, the haptic feedback unit 15 may be worn by the user 16, such as in the form of a glove, band, clothing etc.

In the claims, any reference signs placed between parentheses shall not be construed as limiting the claim. The word "comprising" does not exclude the presence of elements or steps other than those listed in a claim. The word "a" or "an" preceding an element does not exclude the presence of a plurality of such elements. The invention may be implemented by means of hardware comprising several distinct elements, and/or by means of a suitably programmed processor. In the device claim enumerating several means, several of these means may be embodied by one and the same item of hardware. Measures recited in mutually different dependent claims may advantageously be used in combination. Hardware, software and/or method embodiments of the invention may advantageously be used in combination.

### Reference signs

- 11: Imaging accessory positioning system
- 12: Imaging accessory
- 121: Position of imaging accessory
- 122: Reference position
- 13: Position sensor
- 131: Position signal
- 14: Processing unit
- 141: Feedback signal
- 15: Haptic feedback unit
- 151: Haptic feedback
- 16: User
- 17: Imaging apparatus
- 18: User interface
- 21: Inertial sensor
- 211: Movement signal
- 22: Touch sensor
- 221: Touch signal
- 300: Method for positioning of imaging accessory
- S1: Receiving a position signal
- S1': Automatically determining reference position
- S2: Compare the position of the imaging accessory to a reference position
- S3: Computing a feedback signal
- S4: Sending the feedback signal

## Claims

1. An imaging accessory positioning system (11) for medical imaging, the system comprising:
an imaging accessory (12) configured to be positioned relative to a reference position (122),
a position sensor (13) configured to measure a position (121) of the imaging accessory (12), and provide a position signal (131) being indicative of the position (121) of the imaging accessory (12),
a processing unit (14) configured to receive, store and/or determine the reference position (122), derive the position (121) of the imaging accessory (12) from the position signal (131), compare the position (121) of the imaging accessory (12) to the reference position (122), and compute a feedback signal (141) based on the deviation between the position (121) of the imaging accessory (12) and the reference position (122), and
a haptic feedback unit (15) configured to provide haptic feedback (151) to a user (16) in response to the feedback signal (141), and
wherein the haptic feedback (151) is configured to assist the user (16) with positioning of the imaging accessory (12) relative to the reference position (121).

2. The system according to claim 1, wherein the haptic feedback unit (15) is located on or integrated with the imaging accessory (12).

3. The system according to claim 1 or 2, wherein the haptic feedback unit (15) comprises a touch sensor (22) configured to detect touching of the haptic feedback unit (15) and/or an inertial sensor (21) configured to detect movement of the haptic feedback unit (15).

4. The system according to claim 3, wherein the haptic feedback unit (15) is configured to provide haptic feedback (151) via haptic actuators configured to interact with a user (16) touching the haptic feedback unit (15), and wherein the haptic feedback unit (15) is configured to detect a position where the user (16) touches the haptic feedback unit (15) and provide haptic feedback (151) at that position.

5. The system according to any of the preceding claims, wherein a characteristic of the haptic feedback 151 is automatically adapted to a stage of an imaging workflow.

6. The system according to any of the preceding claims, wherein the position sensor (13) is a LIDAR or RADAR or 3D camera sensor, and the position sensor (13) is configured to generate at least one image of the imaging accessory (12) and a patient.

7. The system according to claim 6, wherein the processing unit (14) is configured to process at least one image from the position sensor (13) and automatically determine a reference position (122) relative to the patient.

8. A medical imaging system comprising the imaging accessory positioning system (11) as claimed in any of the preceding claims.

9. A method (300) of positioning an imaging accessory (12) for medical imaging, the method (300) comprising:
Receiving (S1) a position signal (131) from a position sensor (13) configured to measure a position (121) of the imaging accessory (12),
processing (S2) the position signal (131) to compare the position (121) of the imaging accessory (12) to a reference position (122),
computing (S3) a feedback signal (141) based on the deviation of the position (121) of the imaging accessory (12) and the reference position (122), and
sending (S4) the feedback signal (141) to a haptic feedback unit (15), wherein the feedback signal (141) is configured to trigger the haptic feedback unit (15) to generate haptic feedback (151) to a user (16), and
wherein the haptic feedback (151) triggered by the feedback signal (141) is configured to assist the user (16) with positioning of the imaging accessory (12) relative to the reference position (122).

10. The method (300) according to claim 9, wherein the processed position signal (131) includes an image of the imaging accessory (12) and a patient.

11. The method (300) according to claim 10, wherein the reference position (122) is automatically determined (S1') relative to the position of the patient.

12. A computer program element, which, when being executed by at least one processing unit (14), is adapted to cause the processing unit (14) to perform the method according to claim 9 or 10 or 11.

13. A computer readable medium having stored thereon the program element of claim 12.

14. An imaging accessory (12) configured to be used in an imaging accessory positioning system (11) for medical imaging, according to claim 2.
